(19) Europäisches Patentamt
European Patent Office
Office européen des brevets

(11) EP 1 038 870 A1

(12) **EUROPEAN PATENT APPLICATION**

(43) Date of publication:
**27.09.2000 Bulletin 2000/39**

(21) Application number: **00400796.9**

(22) Date of filing: **23.03.2000**

(51) Int Cl.[7]: **C07D 311/92**, C07D 405/06, C07C 235/66, G02B 5/23, C09K 9/02

(84) Designated Contracting States:
**AT BE CH CY DE DK ES FI FR GB GR IE IT LI LU MC NL PT SE**
Designated Extension States:
**AL LT LV MK RO SI**

(30) Priority: **24.03.1999 RU 99106203**

(71) Applicant: **Corning S.A.**
**77920 Samois sur Seine (FR)**

(72) Inventors:
- **Arsenov, Vladimir Dmitrievich**
  **Moscow Region 141700 (RU)**
- **Gorelik, Alexandr Mikhailovich**
  **Moscow 117574 (RU)**
- **Barachevsky, Valery Alexandrovich**
  **Moscow 111672 (RU)**
- **Alfimov, Mikhail Vladimirovich**
  **Moscow 109072 (RU)**

(74) Representative: **Le Roux, Martine et al**
**Cabinet Beau de Loménie**
**158, rue de l'Université**
**75340 Paris Cédex 07 (FR)**

(54) **3,3-bis(aryl)-5-((N-(un)substituted)amido)naphthopyrans, their preparation, compositions and (co)polymer matrices containing them**

(57) The object of the present invention is novel naphthopyran compounds as well as the compositions and (co)polymer matrices containing them. Said compounds have interesting photochromic properties. Another object of the present invention is a method of preparing said novel compounds.

EP 1 038 870 A1

## Description

The present invention relates to novel naphthopyran compounds, more particularly 3,3-bis(aryl)-5-[N-(un) substituted)amido]naphthopyrans, which have, in particular, photochromic properties. The invention also relates to:

- the preparation of such compounds ;
- photochromic compositions and photochromic ophthalmic articles (lenses for example) which contain said naphthopyrans ; and
- intermediate compounds useful in their preparation.

The photochromic compounds are capable of changing colour under the influence of a poly- or monochromatic light (UV for example) and of returning to their initial colour when the luminous irradiation ceases, or under the influence of temperature and/or a poly- or monochromatic light different from the first.

The photochromic compounds find applications in various fields, e. g. for the manufacture of ophthalmic lenses, contact lenses, solar protection glasses, filters, camera optics or photographic apparatus optics or other optical devices and observation devices, glazing, decorative objects, bill elements or even for information storage by optical inscription (coding).

In the field of ophthalmic optics, and in particular the spectacles trade, a photochromic lens which comprises one or more photochromic compounds must have:

- a high transmission in the absence of ultraviolets,
- a low transmission (high colorability) under solar irradiation,
- adapted coloration and discoloration kinetics,
- a tint acceptable to the consumer (grey or brown preferably) with preferably a maintenance of the chosen tint during the coloration and the discoloration of the lens,
- a maintenance of the performances, the properties, within a temperature range of 0-40°C,
- a significant durability, since these objectives sought after are sophisticated corrective lenses and therefore expensive.

These lens characteristics are in fact determined by the active photochromic compounds which they contain; compounds which must furthermore be perfectly compatible with the organic or inorganic support which constitutes the lens.

Moreover, it is to be noted that obtaining a grey or brown tint may necessitate the use of at least two photochromes of different colours, i. e. having distinct maximal absorption wavelengths in the visible. This association further imposes other requirements of the photochromic compounds. In particular, the coloration and discoloration kinetics of the (two or more) associated active photochromic compounds must be essentially identical. The same applies for their stability with time and also for their compatibility with a plastic or inorganic support.

US-A-5,244,602 (PPG Industries, Inc.) describes the first syntheses and applications of novel reversible photochromic naphthopyran compounds of formula :

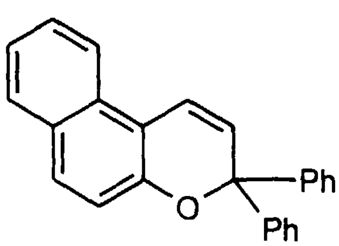

,

also described in this US patent were organic host materials that contain or that are coated with such compounds.

Numerous other patents on naphthopyrans have since been published. US-A-5,637,262 is one of the more recent documents to be published. It describes reversible photochromic 3H-naphtho[2,1-b]pyran compounds of formula :

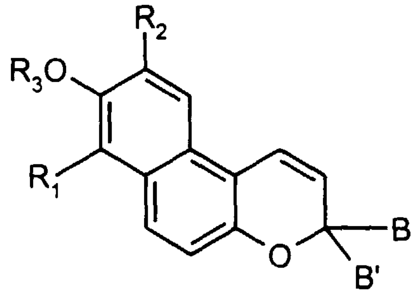

in which $R_1$ is hydrogen or alkyl, $R_2$ is hydrogen or preferably a carboalkoxy group and $R_3$ is hydrogen or preferably an alkyl group, provided that either $R_1$ or $R_2$ is hydrogen, and B and B' are the aryl groups phenyl or naphthyl, a heterocyclic aromatic group or together form a spiro adamantylene group.

It is to the credit of the Applicant to have discovered a novel type of naphthopyran compound which possesses particularly advantageous photochromic properties. It is also to the credit of the Applicant to have proposed a means of access (an efficient synthesis method) of preparing such novel naphthopyran compounds. More specifically, said compounds possess high $\lambda_{max}$ and a high colorability, associated with rapid discoloration kinetics.

Thus, the object of the present invention is novel compounds of formula (I):

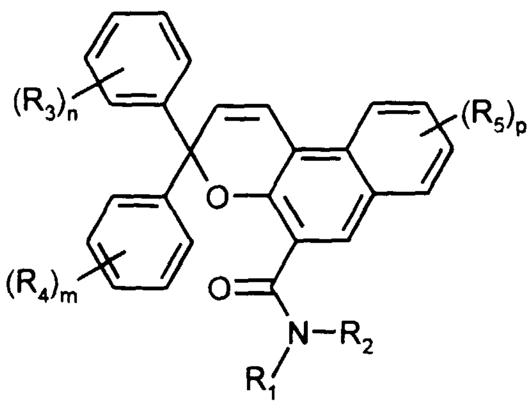

(I)

in which :

- $R_1$ and $R_2$, identical or different, independently represent:
  - hydrogen,
  - a linear alkyl group having 1 to 4 carbon atoms, advantageously 1 or 2 carbon atoms,
  - a cycloalkyl group having 3 to 12 carbon atoms,
  - an aryl or aryloxy group having 6 to 24 carbon atoms in its basic structure, or a heteroaryl or heteroaryloxy group having 4 to 24 carbon atoms in its basic structure and at least one heteroatom selected from sulphur, oxygen and nitrogen ; said basic structures optionally being substituted with at least one substituent selected from :
    - + a halogen, notably fluorine, chlorine or bromine,
    - + a linear or branched alkyl group having 1 to 6 carbon atoms,
    - + a linear or branched alkoxy group having 1 to 6 carbon atoms,
    - + a linear or branched haloalkyl or haloalkoxy group having 1 to 6 carbon atoms, and notably a fluoroalkyl group of this type, advantageously a trifluoromethyl group,

+ a linear or branched alkenyl or alkynyl group having 2 to 12 carbon atoms and notably a vinyl group or an allyl group,
+ an $-NO_2$ group,
+ an $-NH_2$ group,
+ an -NHR group, R representing a linear or branched alkyl group having 1 to 6 carbon atoms,
+ a

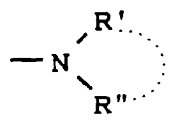

group, R' and R", identical or different, representing independently a linear or branched alkyl group having 1 to 6 carbon atoms or representing, together with the nitrogen atom to which they are bound, a 5- to 7-membered ring which can comprise at least one other heteroatom selected from oxygen, sulphur and nitrogen, said 5- to 7-membered ring being non-substituted or substituted with an R"' group, said R"' group being selected from a linear or branched alkyl group having 1 to 6 carbon atoms, an amine group, and a nitro group; in the case where said other heteroatom is nitrogen, said nitrogen may optionally be substituted with a linear or branched alkyl group having 1 to 6 carbon atoms,
+ a methacryloyl group or an acryloyl group,
+ an epoxy group of formula :

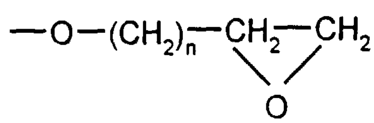

in which n = 1, 2 or 3,

- an aralkyl, aryloxyalkyl, heteroaralkyl or heteroaryloxyalkyl group, the linear or branched alkyl group having 1 to 4 carbon atoms and the aryl, aryloxy, heteroaryl, and heteroaryloxy groups having the definitions given above,
  or
- said two substituents $R_1$ and $R_2$ together form with the N-atom to which they are bound, a 5- to 7-membered ring which can comprise at least one other heteroatom selected from oxygen, sulphur and nitrogen, said 5- to 7-membered ring being non-substituted or substituted with an R"' group, said R"' group being selected from a linear or branched alkyl group having 1 to 6 carbon atoms, an amine group, and a nitro group ; in the case where said other heteroatom is nitrogen, said nitrogen may optionally be substituted with a linear or branched alkyl group having 1 to 6 carbon atoms ; said two substituents $R_1$ and $R_2$, together with the N-atom to which they are bound, representing advantageously a morpholino, piperidino, piperazino, or an indolino group ;

• $R_3$, $R_4$, and $R_5$ identical or different, independently represent:

- a halogen, notably fluorine, chlorine or bromine,
- a linear or branched alkyl group having 1 to 12 carbon atoms (advantageously 1 to 6 carbon atoms),
- a cycloalkyl group having 3 to 12 carbon atoms,
- a linear or branched alkenyl or alkynyl group having 2 to 12 carbon atoms,
- a linear or branched alkoxy or alkylthio group, having 1 to 12 carbon atoms (advantageously 1 to 6 carbon atoms),
- a haloalkyl, a halocycloalkyl or haloalkoxy group corresponding respectively to the alkyl, cycloalkyl, alkoxy groups above, substituted with at least one halogen atom, notably selected from fluorine, chlorine and bromine,
- an electron-withdrawing group, such as a CN, $NO_2$ or SCN group,
- an aryl, aryloxy, heteroaryl or heteroaryloxy group having the same definition as that given above for $R_1$, $R_2$,
- an aralkyl, aryloxyalkyl, heteroaralkyl or heteroaryloxyalkyl group, the linear or branched alkyl group having 1 to 4 carbon atoms and the aryl, aryloxy, heteroaryl, and heteroaryloxy groups having the same definitions as those given above for $R_1$, $R_2$,

- an amino or amido group : $-NH_2$,- NHR, - $CONH_2$,- CONHR,

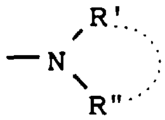 or 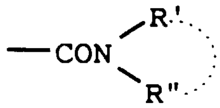 ;

R, R', R" having their respective definitions given above for the amine substituents of the $R_1$, $R_2$ values: aryl or heteroaryl,
- an - $OCOR_6$ or $-COOR_6$ group, $R_6$ representing a hydrogen atom, or a straight or branched alkyl group having 1 to 6 carbon atoms or a cycloalkyl group having 3 to 6 carbon atoms or a phenyl group optionally substituted with at least one of the substituents listed above for the $R_1$, $R_2$ values: aryl, aryloxy, heteroaryl or heteroaryloxy ; or
- in the case in which m is equal to or greater than 2 in $(R_4)_m$, and/or in the case in which n is equal to or greater than 2 in $(R_3)_n$, and/or in the case where p is equal to or greater than 2 in $(R_5)_p$, said radicals, being preferably borne by two adjacent carbon atoms, can combine to form at least one aromatic ring having 5 or 6 members, or an aliphatic ring having 5 to 7 members, preferably 5 or 6 members, said ring(s) comprising, optionally, at least one heteroatom selected from sulphur, oxygen and nitrogen, so as to form at least one heterocyclic ring, the latter being optionally substituted by one or more radicals, which may be identical or different, and have the same definition as given above for $R_3$, $R_4$ and $R_5$ ; and

• m, n, and p are independently an integer from 0 to 5.

As regards $R_3$ and $R_4$, it must be understood that they represent, respectively, optional substituent(s) on the phenyl rings borne in position 3 of the naphthopyran, wherein each of said phenyl rings there are five possible independently substitutable positions ; as regards $R_5$, it must be understood that it represents optional substituent(s) on the naphthyl moiety of the naphthopyran, wherein said naphthyl moiety there are five optionally substitutable positions. Said substituents may exist independently (i.e. at least one of m , n and p $\neq$ 0) or do not exist at all (i.e. at least one of n , m and p = 0). When at least two substituents $R_3$, $R_4$ and $R_5$ do exist, they may be identical or different.
Preferred compounds of the present invention are those of formula (I'):

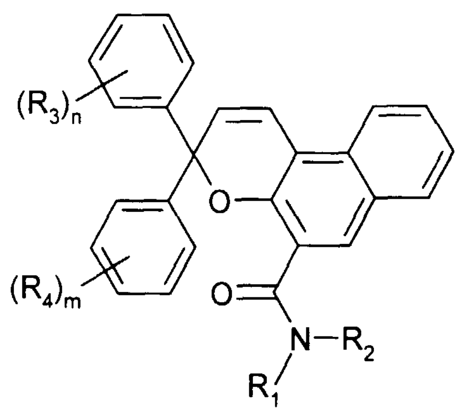

(I')

in which $R_1$, $R_2$, $R_3$, $R_4$, m, and n are as defined for formula (I).
Particularly preferred compounds of the present invention are those of formula (I) or (I') in which one of $R_1$ and $R_2$ is an unsubstituted or substituted aryl group, advantageously an unsubstituted or substituted phenyl group.
The compounds of formula (I) or (I') which are more preferred still are selected from the group consisting of the following : 3,3-diphenyl-5-p-tolylcarbamoyl-3H-naphtho[2,1-b]pyran

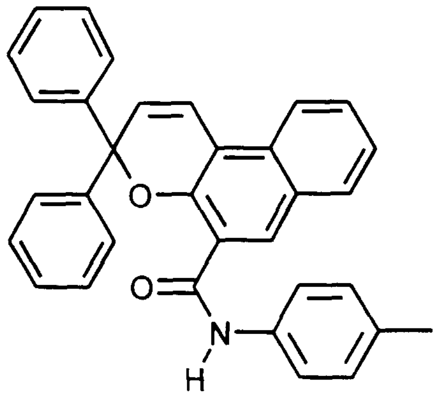

3,3-bis(2,4-dimethoxyphenyl)-5-morpholinocarbonyl-3H-naphtho[2,1-b]pyran

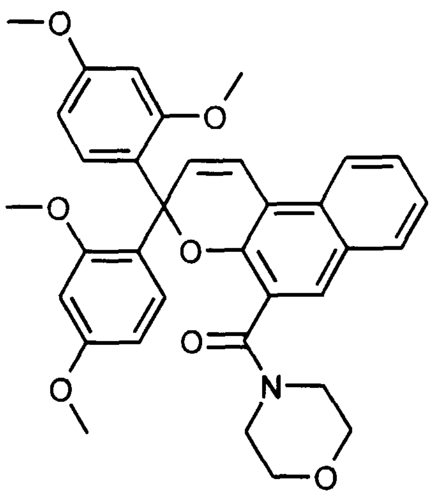

3,3-bis(4-methoxyphenyl)-5-p-tolylcarbamoyl-3H-naphtho-[2,1-b]pyran

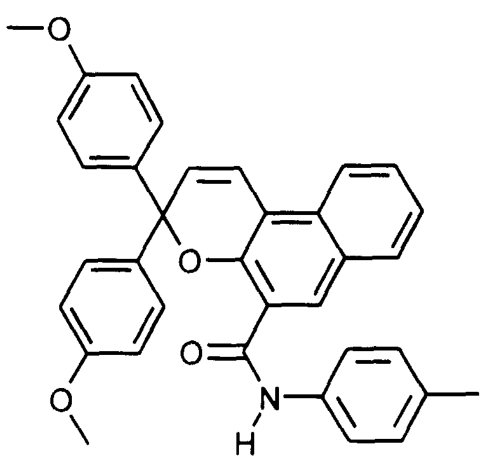

3-(4-dimethylaminophenyl)-3-(4-dimethylaminonapthyl)-5-morpholinocarbonyl-3H-naphtho[2,1-b]pyran

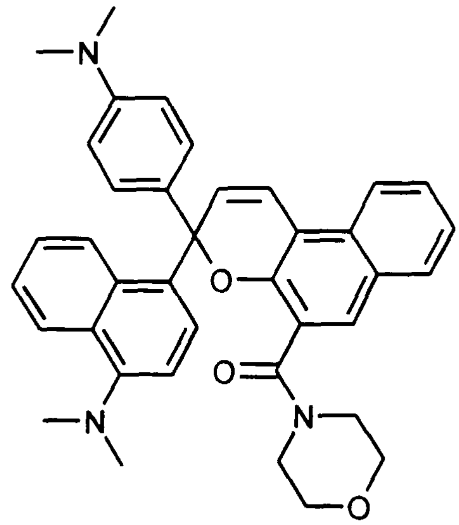

3,3-bis(2,4-dimethoxyphenyl)-5-indolinocarbonyl-3H-naphtho[2,1-b]pyran

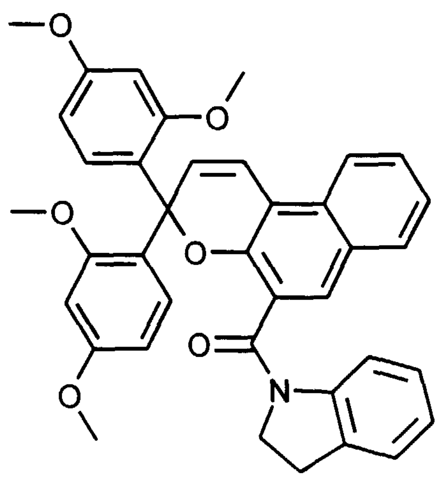

3-(2,4-dimethoxyphenyl)-3-(4-methoxyphenyl)-5-morpholinocarbonyl-3H-naphtho-[2,1-b]pyran

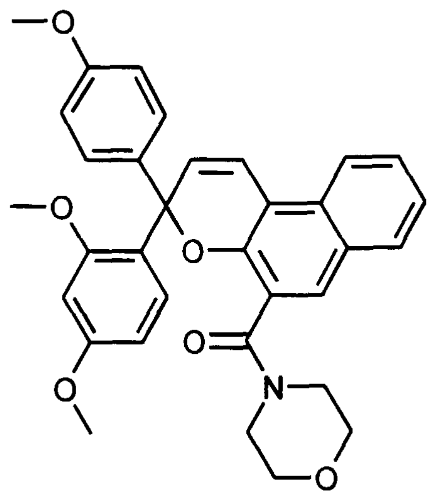

3-(2,4-dimethoxyphenyl)-3-(3,4-dimethoxyphenyl)-5-morpholinocarbonyl-3H-naphtho[2,1-b]pyran

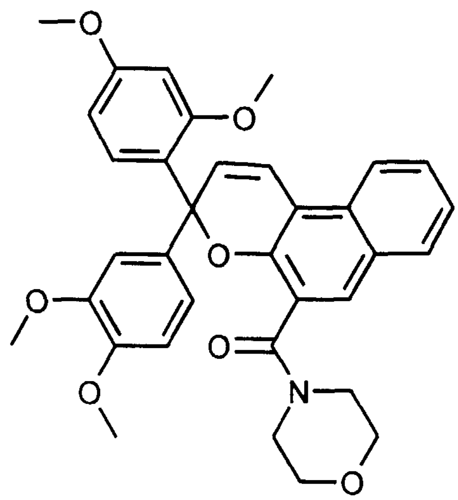

3-(2,4-dimethoxyphenyl)-3-(p-N-piperidinophenyl)-5-piperidinocarbonyl-3H-naphtho[2,1-b]pyran

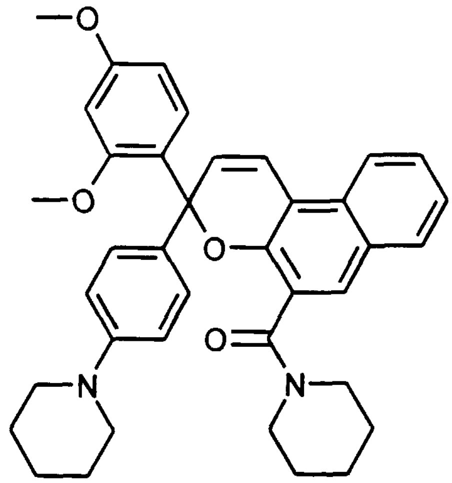

3,3-bis(2,4-dimethoxyphenyl)-5-piperidinocarbonyl-3H-naphtho[2,1-b]pyran

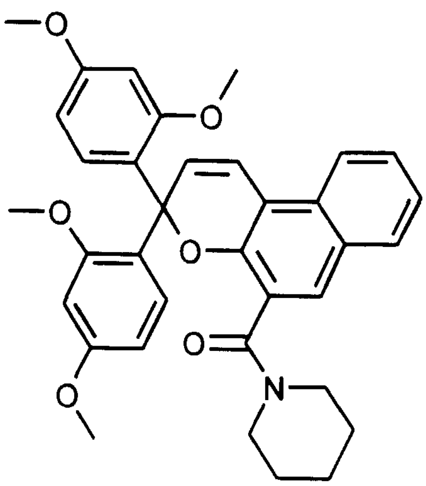

Amongst the substituents ($R_1$, $R_2$, $R_3$, $R_4$ and $R_5$) of the compounds of the invention, there are some which comprise and/or form at least one polymerizable and/or cross-linkable reactive group. The presence of such reactive groups can prove to be advantageous. Thus, the present invention includes, in its first object, naphthopyran compounds, such as defined above, whose structure includes at least one polymerisation and/or cross-linking reactive group; said group consisting of an alkenyl or alkynyl group, advantageously vinyl or allyl, or of a methacryloyl, acryloyl or epoxy group.

Thus, the compounds of the invention which belong to this class can be grasped as monomers, of different nature or not, which can react with themselves and/or with other co-monomers in order to form homopolymers and/or copolymers which are carriers of a photochromic functionality (insofar as said monomers of the invention bear said photochromic functionality) and possess the mechanical properties of macromolecules.

It follows that another object of the present invention is formed by these linear or branched homopolymers or copolymers, at least in part constituted by the compounds of the invention.

Similarly, the above-mentioned compounds of the invention can be envisaged as cross-linking agents having reactive functions which can allow bridges between chains of photochromic or non-photochromic polymers. The reticulates (products of cross-linking) which can be obtained also constitute another object of the present invention.

The compounds of the invention - 3,3-bis(aryl)-5-[(N-(un)substituted)-amido]naphthopyrans of formula (I) -

can be obtained in a general manner by : a) allowing an acetylide salt of formula (II) :

$$\equiv^{-} M^{+} \qquad \text{(II)}$$

in which M represents a metal, to react with a benzophenone of formula (III):

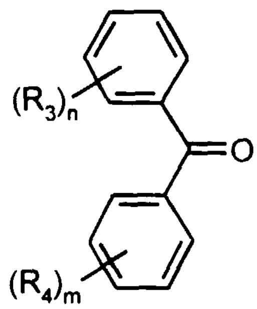

(III)

in which $R_3$, $R_4$, n , and m are as defined above, to provide a propynol of formula (IV) :

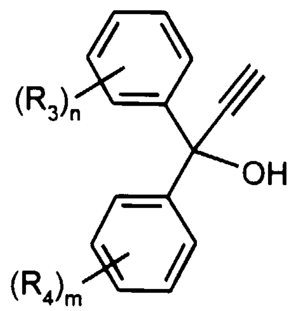

(IV)

in which $R_3$, $R_4$, n, and m are as defined above, which is then allowed to react with a 2-hydroxy-3-naphthamide of formula (V):

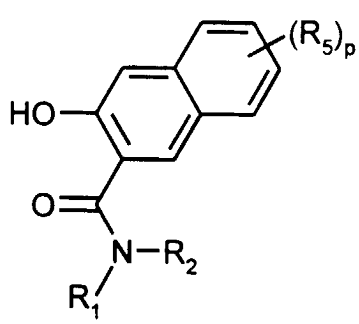

(V)

in which $R_1$, $R_2$, $R_5$, and p are as defined above, to provide a compound of formula (I):

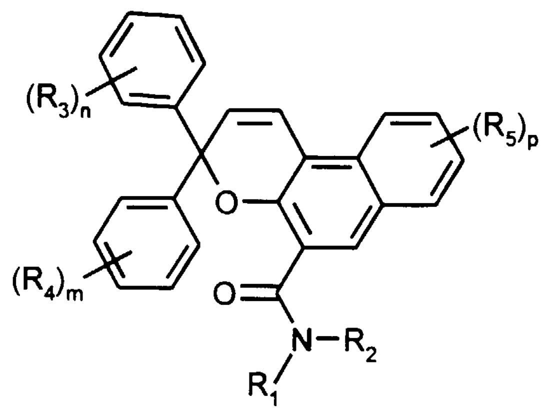

(I)

in which $R_1$, $R_2$, $R_3$, $R_4$, $R_5$, n, m, and p are as defined above.

This method of preparation constitutes another aspect of the presently claimed invention.

This method encompasses the preparation of compounds of formula (V).

Said compounds of formula (V) are obtained according to an original synthesis scheme whose various steps are known to the person skilled in the art, or are adapted from the literature. Said compounds of formula (V) are also novel and thus constitute a further aspect of the present invention.

It is to the credit of the Applicant to have prepared and tested the original naphthopyran compounds of formula (I) described above; said compounds possess particularly advantageous photochromic properties. More specifically, these novel compounds possess a high colorability, with higher $\lambda$max values than the known 3,3-bis(aryl)naphthopyran compounds of analogous structure tested: 3,3-bisphenyl-3H-naphtho[2,1-b]pyran (Control).

Furthermore, these compounds are compatible with the organic polymer or inorganic material support matrices, both in the form included in said matrices and in the form of a coating of said matrices.

In solution or in a polymer matrix, the compounds according to the invention are colourless or faintly coloured in the initial sate and rapidly develop an intense coloration under UV light (365 nm) or a light source of the solar type. Finally, they regain their initial coloration when the irradiation ceases.

According to another of its objects, the present invention relates to the use of said compounds of formula (I) of the invention as photochromic agents. In other words, the Applicant presently proposes:

- novel photochromic compounds which consist of the 3,3-bis(aryl)naphthopyran derivatives such as defined above, taken alone or in a mixture of themselves and/or with at least one other photochromic compound of another type and/or with at least one non-photochromic colouring agent;
- novel photochromic compositions which comprise at least one 3,3-bis(aryl)naphthopyran derivative such as defined above and/or at least one (co)polymer and/or reticulate having at least one of said 3,3-bis(aryl)naphthopyran derivatives of the invention in its structure. Such photochromic compositions can contain at least one other photochromic compound, of another type and/or at least one non-photochromic colouring agent and/or at least one stabilising agent.

Said photochromic compounds of another type, non-photochromic colouring agents, and stabilising agents are prior art products known to the person skilled in the art.

Combinations of photochromic compounds of the invention and/or photochromic compounds of the invention and photochromic compounds of another type according to the prior art are particularly recommended which are suitable for generating grey or brown tints.

The compounds of the invention, notably as photochromic compounds, can be used in solution. Thus, a photochromic solution can be obtained by dissolving at least one of said compounds in an organic solvent such as toluene, dichloromethane, tetrahydrofuran or ethanol. The solutions obtained are in general colourless and transparent. When exposed to sunlight, they develop a high coloration and regain the colourless state when they are placed in an area of less exposure to the sun's rays or, in other words, when they are no longer submitted to UV. In general, a very low concentration of product (of the order of 0.01 to 5% by weight) is sufficient to obtain an intense coloration.

The compounds of the invention (3,3-bis(aryl)naphthopyran derivatives of formula (I)) can also be used as a

photochromic material dispersed uniformly in the mass or on the surface of a polymer matrix. In fact, the most interesting applications of the compounds of the invention are those in which the photochrome is dispersed uniformly within or on the surface of a polymer, copolymer or mixture of polymers. The (co)polymer matrix which comprises said photochrome of the invention (at least one, in a free form, and/or in the form of a (co)polymer and/or reticulate, and/or in the form of a photochromic composition, such as defined above) constitutes another object of the present invention.

The methods of implementation which can be envisaged in order to obtain such a matrix are very varied. Amongst those known to the person skilled in the art, the diffusion in the (co)polymer, from a suspension or solution of the photochrome, in a silicone oil, in an aliphatic or aromatic hydrocarbon, or in a glycol, or from another polymer matrix, can be cited for example. The diffusion is commonly carried out at a temperature of 50 to 200°C for a period of time of 15 minutes to several hours, according to the nature of the polymer matrix. Another implementation technique consists in mixing the photochrome in a formulation of polymerizable matrices, depositing this mixture on a surface or in a mould, and then carrying out the copolymerisation. These implementation techniques , and others, are described in the article by Crano *et al.* "Spiroxazines and their use in photochromic lenses" published in Applied Photochromic Polymer Systems, Ed. Blackie and Son Ltd - 1992.

In accordance with a variant of this object of the invention, it is also envisagable to graft the photochromes onto the (co)polymers. Thus, the invention also relates to the (co)polymers grafted by at least one of the photochromes described above. Thus, the expression "(co)polymer matrix comprising at least one photochrome of the invention" means both matrices which comprise said photochrome in their mass and on their surface, and matrices grafted by said photochrome.

The following products can be mentioned as examples of polymeric materials preferred for optical applications of the photochromic compounds according to the invention:

- optionally halogenated alkyl, cycloalkyl, aryl or aralkyl poly[mono-, di-, tri-, tetra-]acrylate or poly[mono-, di-, tri-, tetra-]methacrylate or having at least one ether and/or ester and/or carbonate and/or carbamate and/or thiocarbamate and/or urea and/or amide group,
- polystyrene, polyether, polyester, polycarbonate (e. g. bisphenol-A polycarbonate, diallyl diethylene glycol polycarbonate), polycarbamate, polyepoxy, polyurea, polyurethane, polythiourethane, polysiloxane, polyacrylonitrile, polyamide, aliphatic or aromatic polyester, vinylic polymers, cellulose acetate, cellulose triacetate, cellulose acetate-propionate or polyvinylbutyral,
- copolymers of at least two types of co-polymerizable monomers selected from precursor monomers of the polymers listed above (notably selected from (meth)acrylics, vinyls, allyls, and mixtures thereof).

Resins which are specifically described in French patent application No. 97 05458 of the 2$^{nd}$ May 1997 (to be published under No. 2 762 845 on the 6$^{th}$ of November 1998) may be especially mentioned.

The photochromic compounds according to the invention can be used alone or in a mixture with other products in order to form a composition which can be a solid or a liquid, in solution or in suspension for example, as has already been indicated above. These compositions, which constitute an object of the invention as already indicated above, can therefore comprise the compounds of the invention and other additional photochromic compounds enabling obtaining dark colourations, grey or brown for example, desired by the public in applications such as ophthalmic or solar spectacles trade. These additional photochromic compounds can be those known to the person skilled in the art and described in the literature, e. g. chromenes (US-A-3,567,605, US-A-5,238,981, WO-A-94 22850, EP-A 562 915), spiropyrans or naphthospiropyrans (US-A-5,238,981) and spiroxazines (Crano et *al.,* "Applied Photochromic Polymer Systems", Ed. Blackie & Son Ltd, 1992, chapter 2 ).

Said compositions according to the invention can also comprise :

- non-photochromic colouring agents which enable adjusting the tint,
- and/or one or more stabilising agents, such as an anti-oxidising agent for example,
- and/or one or more anti-UV,
- and/or one or more anti-radicals,
- and/or one or more photochemical excited state deactivators.

These additives can notably enable improving the durability of said compositions.

According to another of its aspects relative to the application of the compounds of the invention, another object of the present invention is ophthalmic articles, such as articles for the ophthalmic and solar spectacles trade, which comprise at least one compound according to the invention and/or at least one (co)polymer and/or reticulate formed, at least in part, from compound(s) of the invention and/or at least one composition containing at least one compound of the invention and/or at least one matrix, such as defined above, of an organic polymer material or an inorganic material or even of an inorganic-organic hybrid material incorporating therein at least one compound of the

invention.

In practice, the articles most particularly covered by the present invention are photochromic ophthalmic or solar lenses, glazing (window panes for buildings, locomotion engines, automobiles), optical devices, decorative devices, solar protection devices, information storage, etc.

The present invention is illustrated by the following Examples of synthesis and photochromic validation, of the compounds of the invention (3,3-bis(aryl)naphthopyrans). Said compounds of the invention are compared to the prior art compound 3,3-bisphenyl-3H-naphtho[2,1-b]pyran (Control).

**EXAMPLES**

GENERAL:

$^1H$ NMR spectra were recorded in $d_6$-DMSO, $CD_3CN$ and $CDCl_3$ on a Brüker AC-200p and Brüker AMX-400 spectrometers using tetramethylsilane as internal standard.
Mass spectra were obtained on a Varian MAT 311A mass spectrometer at an ionisation energy of 70 eV with a direct insertion of samples into the ionisation chamber.
The progress of the reactions was monitored by TLC on DC-Alufolien Kieselgel-60 $F_{254}$ plates (from Merck).

**Example 1 : 3,3-diphenyl-5-p-toiylcarbamoyl-3H-naphtho(2,1-b]pyran.**

Formula (I) : $R_1$ = H, $R_2$ = p-tolyl, m = n = p = 0.

Step 1a : 2-hydroxy-3-p-tolylcarbamoylnaphthalene.

A mixture of 2-hydroxy-3-naphthoic acid (10.0g, 0.053 mole), p-methylaniline (5.75g, 0.053 mole), urea (0.5g) and 65 ml of toluene was warmed to 75°C, and phosphorus trichloride (3.2g, 0.032 mole) was added dropwise over 2 hours. The reaction mixture was heated for an additional 2 hours at 80-85°C and was refluxed for 8 hours. The reaction mixture was then cooled to 90-100°C and was transferred into a solution of sodium carbonate (3g) in water (27ml). The toluene was evaporated off with steam, the resulting suspension was filtered, and the precipitate was washed with hot water, dried and crystallised using ethyl acetate. The light-pink powder of 2-hydroxy-3-p-tolylcarbamoylnaphthalene (12.3g) was produced.

Step 1b : 1,1-Biphenyl-2-propyn-1-ol.

Acetylene was bubbled through a mixture of powdered potassium hydroxide ( 25 g, 0.45 mole) and 125 ml tetrahydrofuran at 14-16°C for 1 hour and a solution of benzophenone (21 g, 0.115 mole) in 50 ml tetrahydrofuran was added dropwise at 30-40°C for 1 hour. The resulting reaction mixture was stirred for 1 hour under an acetylene atmosphere at the same temperature, transferred into 700 ml of water, and extracted with ethyl acetate. The extract was dried over anhydrous sodium sulphate, and the solvent removed *in vacuo.* The resultant oil was dissolved in an ether-hexane mixture and after cooling, the white powder was filtered off. The product (21.5 g) melted at 47°C and was 1,1-diphenyl-2-propyn-1-ol.

Step 1c : 3,3-diphenyl-5-p-tolylcarbamoyl-3H-naphtho[2,1-b]pyran.

To a hot solution of 2-hydroxy-3-p-tolylcarbamoylnaphthalene (0.56g, 0.002 mole) in 80 ml of benzene was added 1,1-diphenyl-2-propyn-1-ol (0.42 g, 0.002 mole) and after that acidic alumina (0.2 g) was added. The reaction mixture was stirred and refluxed for 3 hours, filtered, and 60 ml of solvent was removed. After cooling, the light-grey powder (0.74 g) was filtered off. The NMR spectrum confirmed the product to be 3,3-diphenyl-5-p-tolylcarbamoyl-3H-naphtho[2,1-b]pyran.

$^1H$ NMR, DMSO d6, $\delta$: 2.20 (s, 3H, Me), 6.60 (d, 1H, 2-H), 7.93 (d, 1H, 6-H), 10.13(s, 1H, NH).

**Example 2 : 3,3-bis(2,4-dimethoxyphenyl)-5-morpholinocarbonyl-3H-naphtha[2,1-b]pyran**

Formula (I) : $R_1$ and $R_2$ with the nitrogen atom to which they are attached represent N-morpholino, m = n = 2, $R_3$ = $R_4$ = 2,4-dimethoxy , p = 0.

Step 2a: 2,2',4,4'-tetramethoxybenzophenone.

A mixture of 2,2'-dihydroxy-4,4'-dimethoxybenzophenone (18 g, 0.66 mole), dimethyl sulphate (25 ml, 0.264 mole), potassium carbonate (36.4g , 0.264 mole) and 135 ml anhydrous acetone was stirred and refluxed for 23 hours, cooled by pouring it into a beaker containing ice water. The product was filtered off, washed with water, dried and crystallised with activated carbon using ethyl alcohol. 2,2',4,4'-tetramethoxybenzophenone was obtained as white crystals (16.7 g) melting at 138-139°C.

Step 2b : 1,1-bis(2,4-dimethoxyphenyl)-2-propyn-1-ol.

In accordance with procedure of Example 1b, step 2, 2,2',4,4'-tetramethoxybenzophenone in place of benzophenone was reacted with potassium acetylide at 40-45°C. The product was crystallised using an ether-hexane mixture. The yield was quantitative. The product was 1,1-bis(2,4-dimethoxyphenyl)-2-propyn-1-ol.

Step 2c : 2-hydroxy-3-morpholinocarbonylnaphthalene.

In accordance with procedure of Example la, step 1, 2-hydroxy-3-naphthoic acid was reacted with morpholine in the place of p-methylaniline. The product was crystallised using ethyl acetate and it was 2-hydroxy-3-morpholinocarbonylnaphthalene.

Step 2d: 3,3-bis(2,4-dimethoxyphenyl)-5-morpholinocarbonyl-3H-naphtho[2,1-b]pyran

A catalytic amount of para-toluenesulphonic acid (approximately 20.0 mg) was added to a hot solution of 2-hydroxy-3-morpholinocarbonylnaphthalene (0.27g , 0.001 mole) and 1,1-bis(2,4-dimethoxyphenyl)-2-propyn-1-ol (0.33g , 0.001 mole), in 40 ml of toluene. The resulting mixture was stirred at 60°C for 2 hours, cooled and washed with water. The resulting crude oil was column chromatographed on silica using a 1:2 mixture of toluene:ethyl acetate and crystallised using an ether-hexane mixture. The product was light pink powder and was confirmed by NMR spectroscopy to be 3,3-bis(2,4-dimethoxyphenyl)-5-morpholinocarbonyl-3H-naphtho[2,1-b]pyran.

$^1H$ NMR, $CDCl_3$ , δ : 3.10-3.90 (m, 8H, $(CH_2)_4$), 3.70 (s, 6H, $(OMe)_2$), 3.78 (s, 6H, $(OMe)_2$), 6.32 (d, 1H, 2-H), 7.85 (d, 1H, 6-H).

Various other 5-substituted naphthopyran compounds in accordance with the invention were synthesised by processes analogous to those described in Examples 1 and 2. The naphthopyrans obtained together with their NMR data are given below.

**Example 3: 3,3-bis(4-methoxyphenyl)-5-p-tolylcarbamoyl-3H-naphtha[2,1-b]pyran.**

Formula (I) : $R_1$ = H , $R_2$ = p-tolyl , m = n = 1 , p = 0 , $R_3$ = $R_4$ = 4-OMe.

$^1H$ NMR, DMSO-d6 , δ : 2.20 (s, 3H, Me), 3.68 (s, 6H, $(OMe)_2$), 6.60 (d, 1H, 2-H), 7.91 (d, 1H, 6-H), 10.28 (s, 1H, NH).

**Example 4 : 3-(4-dimethylaminophenyl)-3-(4-dimethylaminonapthyl)-5-morpholinocarbonyl-3H-naphtho [2,1-b]pyran**

Formula (I) : $R_1$ and $R_2$ with the nitrogen atom to which they are attached represent morpholino , $R_3$ = 4-$NMe_2$ , $R_4$ = phenyl fused onto position 2,3 , and 4-$NMe_2$, p=0.

A light-grey powder.

$^1H$ NMR, DMSO-d6 , δ : 2.69 (s, 6H, $NMe_2$), 2.77 (s, 6H, $NMe_2$), 3.10-3.70 (m, 8H, $(CH_2)_4$), 6.20 (d, 1 H, 2-H), 7.73 (t, 1-H, 6-H).

**Example 5 : 3,3-bis(2,4-dimethoxyphenyl)-5-indolinocarbonyl--3H-naphtho[2,1-b]pyran.**

Formula (I) : $R_1$ and $R_2$ with the nitrogen atom to which they are attached represent indolino , m = n = 2 , p = 0 , $R_3$ = $R_4$ = 2,4-di OMe.

A catalytic amount of p-toluenesulphonic acid (approximately 10 mg) was added to a solution of 2-hydroxy-3-indolinocarbonylnaphthalene (0.705 g , 2.4 mmole) and bis(2,4-dimethoxyphenyl)-2-propyn-1-ol (0.80 g, 2.4 mmole) in 120 ml of benzene. The resulting mixture was stirred at 60-70°C for 2 hours, and then washed with water. The organic layer was separated, dried over anhydrous sodium sulphate and the solvent removed *in vacuo.* The resultant

oil was chromatographed on silica gel (eluent - benzene-ethyl acetate 4:1), then flash chromatographed on aluminium oxide (eluent - ethyl acetate) and crystallised using ether. A white powder (0.3 g, 24%) was obtained.

$^1H$ NMR, $C_6D_6$, δ : 0.49 (s, 1H, $CH_2$), 2.40 (s, 2H, $CH_2$), 2.85-3.30 (m, 13H, $(OMe)_4$, $CH_2$), 7.80 (d, 1H, 6-H).

**Example 6: 3-(2,4-dimethoxyphenyl)-3-(4-methoxyphenyl)-5-morpholinocarbonyl-3H-naphtho[2,1-b]pyran.**

Formula (I) : $R_1$ and $R_2$ with the nitrogen atom to which they are attached represent morpholino, n = l, m = 2,$R_3$ = 4-OMe, $R_4$ = 2,4-di-OMe, p = 0.

Step 6a: 2,4,4'-trimethoxybenzophenone.

To a reaction flask were added 5.0 g (0.030 mole) of 2,4-dimethoxybenzoic acid, 3.25 g (0.030 mole) of anisole and 30 g of polyphosphoric acid. The reaction mixture was warmed to 60-70°C with stirring for 2 hours and transferred into ice water. The resulting oil was extracted with ethyl acetate, the organic layer was separated, dried, evaporated and the resulting crude product was purified by column chromatography on silica using a 4:1 mixture of toluene and ethyl acetate as eluent. A light-yellow powder (3.5 g, 43%) was produced.

Step 6b : 1-(2,4-dimethoxyphenyl)-1-(4-methoxyphenyl)-2-propyn-1-ol.

To a solution of 2 g (0.022 mole) of lithium acetylide ethylenediamine complex in 10 ml dry dimethyl sulphoxide was added dropwise a solution of 6 g (0.022 mole) of 2,4,4'-trimethoxybenzophenone in 20 ml of dry dimethyl sulphoxide over 1.5 hours at 60°C. The resultant solution was stirred 1 hour at 60°C and was then transferred into cold water. The resulting oil was extracted with ethyl acetate, the organic layer was separated, dried, evaporated. The crude product (a light-yellow oil, 5 g, 77 %) was used without additional purification.

Step 6c : 2-Hydroxy-3-morpholinocarbonylnaphthalene.

To a reaction flask were added 7.0 g (0.037 mole) of 2-hydroxy-3-naphthoic acid, 3.22 g (0.037 mole) of morpholine, 0.35 g (0.0058 mole) of urea and 50 millilitres of toluene. The reaction mixture was warmed to 75°C and 2.27g (0.017 mole) of phosphorus trichloride were added dropwise over 30 minutes. The resultant mixture was allowed to stand for an additional 1.5 hours at 75-80°C, refluxed for 7 hours, cooled to 90- 100°C, and a solution of 1.73 g (0.0165 mole) of sodium carbonate in 20 ml of water was added to the reaction mixture. The toluene was evaporated off with steam, the resulting suspension was filtered, the precipitate was washed with water, warmed to 70-80°C, dried and dissolved in 150 millilitres of ethyl acetate with boiling. This hot solution was filtered through a layer of silica. Three lots of 90 ml of hot ethyl acetate were then passed through the silica. The organic portions were combined, evaporated *in vacuo* to a small volume (about 30-40 ml) and placed at 5°C overnight. The resulting precipitate was filtered off and dried. A white crystalline powder (5.1 g, 53%) was produced.

Step 6d: 3-(2,4-dimethoxyphenyl)-3-(4-methoxyphenyl)-5-morpholinocarbonyl-3H-naphtho[2,1-b]pyran.

A reaction flask was charged with 1.00g (0.004 mole) of 3-hydroxy-2-morpholinocarbonylnaphthalene and 40 ml of dry toluene. The reaction suspension was heated at 70°C until a solution was obtained. To the flask were added 1.5 g of 1-(2,4-dimethoxyphenyl)-1-(4-methoxyphenyl)-2-propyn-1-ol in 5 ml of dry toluene and catalytic amounts of toluenesulphonic acid. The reaction solution was warmed at 60°C for 4 hours. After filtering, the toluene was evaporated off and the resulting crude product was purified by column chromatography on silica using a 4:1 mixture of toluene and ethyl acetate as eluent. The photochromic fraction was evaporated *in vacuo* and a 1:4 mixture of ether and hexane was added to the resultant pink oil. The precipitate was filtered off, washed with 2 millilitres of the same ether:hexane mixture and dried. An NMR analysis of the white powder (0.65 g, 32%) confirmed it to be 3-(2,4-dimethoxyphenyl)-3-(4-methoxyphenyl)-5-morpholinocarbonyl-3H-naphtho-[2,1-b]pyran.

**Example 7: 3-(2,4-dimethoxyphenyl)-3-(3,4-dimethoxyphenyl)-5-morpholinocarbonyl-3H-naphtha[2,1-b]pyran.**

Formula (I) : $R_1$ and $R_2$ with the nitrogen atom to which they are attached represent morpholino , m = n = 2, $R_3$ = 2,4-di-OMe,

$R_4$ = 3,4-di-OMe, p = 0.

Step 7a: 2,4,3',4'-tetramethoxybenzophenone.

In accordance with the procedure for 2,4,4'-trimethoxybenzophenone, 5 g (0.0027 mole) of 3,4-dimethoxybenzoic acid was used in place of 2,4-dimethoxybenzoic acid and 3.35 g (0.0027 mole) of 1,3-dimethoxybenzene was used in place of anisole. The product was produced as a light-yellow powder (5.6 g, 67%).

Step 7b : 1-(2,4-dimethoxyphenyl)-1-(3,4-dimethoxyphenyl)-2-propyn-1-ol.

In accordance with the procedure for 1-(2,4-dimethoxyphenyl)-1-(4-methoxyphenyl)-2-propyn-1-ol, 1.4 g (0.0046 mole) of 2,4,3',4'-tetramethoxybenzophenone was used in place of 2,4,4'-trimethoxybenzophenone. The product was a red oil (0.64 g, 43%) which was used without additional purification.

Step 7c: 3-(2,4-dimethoxyphenyl)-3-(3,4-dimethoxyphenyl)-5-morpholinocarbonyl-3H-naphtho[2,1-b]pyran.

In accordance with the procedure for 3-(2,4-dimethoxyphenyl)-3-(4-methoxyphenyl)-5-morpholinocarbonyl-3H-naphtho[2,1-b]pyran, 0.44 g of 1-(2,4-dimethoxyphenyl)-1-(3,4-dimethoxyphenyl)-2-propyn-1-ol was used in place of 1-(2,4-dimethoxyphenyl)-1-(4-methoxyphenyl)-2-propyn-1-ol. The temperature of the reaction must be 40°C, but not higher. The crude product was purified by column chromatography on aluminium oxide using a 4:1 mixture of toluene and ethyl acetate as eluent. The photochromic fraction was evaporated *in vacuo* and a 1:3 mixture of ether and hexane was added to the resultant pink oil. The precipitate was filtered, washed with 2 ml of the same ether/hexane mixture and dried. An NMR analysis of the light-pink powder (0.20 g, 26%) confirmed it to be 3-(2,4-dimethoxyphenyl)-3-(3,4-dimethoxyphenyl)-5-morpholinocarbonyl-3H-naphtho[2,1-b]pyran.

$^1H$ NMR, DMSO-d6, δ : 2.80-3.40 (m, 4H, $(CH_2)_2$), 3.50-3.80 (m, 16H, $(OMe)_4$, $(CH_2)_2$), 6.4-8.1 (m, 13H, Ar).

**Example 8: 3-(2,4-dimethoxyphenyl)-3-(p-N-piperidinophenyl)-5-piperidinocarbonyl-3H-naphtho[2,1-b]pyran.**

Formula (I) : $R_1$ and $R_4$ with the nitrogen atom to which they are attached represent piperidino , m = 1 n = 2 , $R_3$ = 2,4-di OMe,
$R_4$ = 4-piperidino , p = 0.

Step 8a : 2,4-Dimethoxybenzoyl-4-(N-piperidino)benzene.

Aluminium trichloride (4.92 g, 0.037 mole) was added over 20 minutes to a solution of 2,4-dimethoxybenzoyl chloride (6.8 g, 0.034 mol), N-phenylpiperidine (5.0 g, 0.034 mole) and 75 ml of anhydrous methylene chloride at 0-5°C under a nitrogen atmosphere with stirring. The reaction mixture was stirred at room temperature for 4 hours, transferred into a solution containing two molar aqueous hydrochloric acid (50 ml). The organic layer was washed with water, a solution containing two molar sodium hydroxide, water, and then dried. The organic solvent was removed *in vacuo.* The resultant oil was flash chromatographed on silica gel (eluent-benzene-ethyl acetate 10:1, ethyl acetate) and crystallised using hexane. A light-yellow powder (2.9 g, 23%) was produced.

Step 8b : 1-(2,4-Dimethoxyphenyl)-1-(4-N-piperidinophenyl)-2-propyn-1-ol.

To a solution of 2,4-dimethoxybenzoyl-4-(N-piperidino)benzene (2.9g, 8.9 mmole) in 9 ml anhydrous dimethyl sulphoxide at 60°C with stirring was added lithium acetylide ethylenediamine complex (0.92 g, 8.9 mmol) over 10 minutes. The reaction mixture was stirred for 3 hours at 60-65°C, cooled to room temperature, transferred to a mixture of ice:water, and extracted with methylene chloride. The extract was dried over magnesium sulphate, and the solvent was removed *in vacuo.* The resultant oil was chromatographed on silica gel (eluent - benzene-ethyl acetate 2:1). The product (1.53 g, 49%) was a light-yellow oil.

Step 8c: 3-(2,4-dimethoxyphenyl)-3-(p-N-piperidinophenyl)-5-piperidinocarbonyl-3H-naphtho[2,1-b]pyran.

To a hot solution of 2-hydroxy-3-piperidinocarbonylnaphthalene (1.13 g, 4.4 mmole) and 1-(2,4-dimethoxyphenyl)-1-(4-N-piperidinophenyl)-2-propyn-1-ol (1.53 g, 4.4 mmole) in 100 ml of toluene was added acidic aluminium oxide (0.50 g). The resulting mixture was stirred at 60-70°C over 4 hours, filtered, and the toluene was removed *in vacuo.* The resultant oil was chromatographed on aluminium oxide (eluent - benzene-ethyl acetate 2:1), then chromatographed on silica gel (eluent-ether) and washed with hexane. A light-grey powder (0.120 g, 4%) was obtained.

$^1H$ NMR, DMSO-d6 , δ : 1.0-1.7 (m, 12H, $(CH_2)_6$), 2.8 (s, 2H, $NCH_2$), 3.08 (s, 4H, $N(CH_2)_2$), 3.57 (s, 3H, OMe), 3.70 (d, 5H, OMe, $NCH_2$), 6.42 (d, 1H, 2-H), 7.81 (d, 1H, 6-H).

**Example 9: 3,3-bis(2,4-dimethoxyphenyl)-5-piperidinocarbonyl-3H-naphtho[2,1-b]pyran.**

Formula (I) : $R_1$ and $R_2$ with the nitrogen atom to which they attached represent piperidino, m = n = 2 , $R_3$ = $R_4$ = 2,4-di OMe, p = 0.

A catalytic amount of p-toluenesulphonic acid (approximately 10 mg) was added to a hot solution of 2-hydroxy-3-piperidinocarbonylnaphthalene (0.383 g, 1.5 mmole) and bis(2,4-dimethoxyphenyl)-2-propyn-1-ol (0.493 g, 1.5 mmole) in 40 ml of benzene. The resulting mixture was stirred at 60-70°C for 2 hours, washed with a solution of sodium hydroxide (1.5 g) in 15 ml of water, then with water. The organic layer was separated, dried over anhydrous sodium sulphate and the solvent was removed *in vacuo.* The resultant oil was chromatographed on aluminium oxide (eluent - benzene-ethyl acetate 4:1) and crystallised using ether. A white powder (0.350 g, 41%) was obtained. An NMR spectrum showed the product to have a structure consistent with the title compound.

**Example 10: Preparation of photochromic matrices and lens samples containing the photochromic compounds of the invention.**

The matrix used in accordance with the present invention may be one of two types:

I. MATRIX I:

A homopolymer of the monomer DIACRYL 121 (D121 : tetraethoxylated Bisphenol A dimethylmethacrylate), marketed by Akzo Nobel ; or

II. MATRIX II:

A copolymer comprised of a 50/50 mixture by weight of D121 (as above) and polyethylene glycol dimethacrylate (Molecular weight = 400) (PEGDMA 400).

The lens samples are prepared as follows :

$10^{-5}$ moles of at least one compound of the invention, together with 0.2% by weight of AMBN (2,2'-azobis (2-methylbutyronitrile)) provided by Akzo, are added to 10 g of formulations able to generate cross-linked matrix I or II. The formulations thus prepared are then poured into a lens mould of 2 mm thickness and then cured by heating at 60°C for 6 hours, followed by heating at 80°C for 2 hours to give a lens sample.
The photochromic properties of the lens samples thus obtained were then evaluated as follows :
Said lens sample, containing said photochromic compounds in its mass, is exposed to a UV radiation (source: xenon lamp). The $\lambda_{max}$ values in the visible and the discoloration kinetics are given in the Table I below :

TABLE I:

| Photochromic properties of lens samples according to the invention. | | | | | | |
|---|---|---|---|---|---|---|
| **Compound Example** | **Matrix** | **$T_0$ %** | **$T_{D15}$ %** | **OID** | **$t_{1/2}$ (s)** | **$\lambda_{max}$ (nm)** |
| **1** | I | 87.9 | 42.6 | 0.315 | 266 | 477 |
| **2** | I | 85.7 | 4.3 | 1.303 | 2000 | 514 |
| **4** | I | 65.0 | 30.8 | 0.325 | 1170 | 556 |
| **5** | I | 85.6 | 7.4 | 1.063 | 1420 | 514 |
| | II | 84.0 | 2.0 | 1.634 | 400 | 514 |
| **6** | I | 85.4 | 1.0 | 1.931 | 1128 | 510 |
| | II | 84.0 | 0.6 | 2.146 | 655 | - |
| **7** | I | 88.0 | 15.8 | 0.746 | 560 | 512 |
| | II | 88.1 | 13.8 | 0.805 | 236 | 512 |
| **8** | I | 85.4 | 19.7 | 0.637 | 560 | 570 |
| | II | 85.8 | 14.6 | 0.769 | 306 | 570 |

TABLE I: (continued)

| Photochromic properties of lens samples according to the invention. | | | | | | |
|---|---|---|---|---|---|---|
| **Compound Example** | **Matrix** | **$T_0$ %** | **$T_{D15}$ %** | **OID** | **$t_{1/2}$ (s)** | **$\lambda_{max}$ (nm)** |
| **9** | II | 89.0 | 15.3 | 0.765 | 290 | 504 |
| **Control** | II | 88 | 44 | 0.280 | 266 | 436 |

Control: 3,3-bisphenyl-3H-naphtho[2,1-b]pyran
$T_0$ : the transmission before exposure to UV
$T_{D15}$: the transmission after 15 minutes' exposure to UV
OID : optical induced density ($D_\infty$-$D_0$) [$D_\infty$ is the density after exposure, $D_0$ the density before exposure]
$t_{1/2}$: time of half-darkness which characterises the kinetics of the return to the initial state when at the equilibrium the exposure is cut off.
$\lambda_{max}$: the maximum absorption in the visible spectrum after UV irradiation.

It is clearly seen from the above Table that when the lens samples containing photochromic compounds of the invention (having a bis(aryl) substituent in position 3, and an N-(un)substituted)amido substituent in position 5 of the naphthopyran ring system) are compared to the control lens sample containing a photochromic compound of the prior art, 3,3-bisphenyl-3H-naphtho[2,1-b]pyran, the following may be noted :

i) the lens samples containing photochromic compounds of the invention have higher $\lambda_{max}$ values than those containing prior art photochromic compound;
ii) the lens samples containing photochromic compounds of the invention have higher optical induced densities (OID) than those containing prior art photochromic compound;
iii) the lens samples containing photochromic compounds of the invention have lower transmissions after 15 minutes' exposure to UV ($T_{D15}$) than those containing prior art photochromic compound.

## Claims

**1.** Compounds of formula (I):

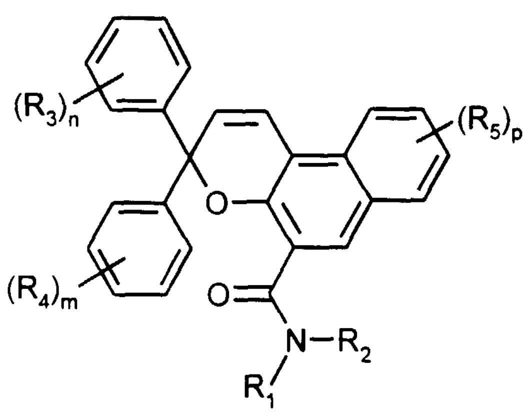

(I)

in which:

- $R_1$ and $R_2$, identical or different, independently represent:
  - hydrogen,
  - a linear alkyl group having 1 to 4 carbon atoms, advantageously 1 or 2 carbon atoms,

- a cycloalkyl group having 3 to 12 carbon atoms,
- an aryl or aryloxy group having 6 to 24 carbon atoms in its basic structure, or a heteroaryl or heteroaryloxy group having 4 to 24 carbon atoms in its basic structure and at least one heteroatom selected from sulphur, oxygen and nitrogen ; said basic structures optionally being substituted with at least one substituent selected from :

  + a halogen, notably fluorine, chlorine or bromine,
  + a linear or branched alkyl group having 1 to 6 carbon atoms,
  + a linear or branched alkoxy group having 1 to 6 carbon atoms,
  + a linear or branched haloalkyl or haloalkoxy group having 1 to 6 carbon atoms, and notably a fluoroalkyl group of this type, advantageously a trifluoromethyl group,
  + a linear or branched alkenyl or alkynyl group having 2 to 12 carbon atoms and notably a vinyl group or an allyl group,
  + an $-NO_2$ group,
  + an $-NH_2$ group,
  + an -NHR group, R representing a linear or branched alkyl group having 1 to 6 carbon atoms,
  + a

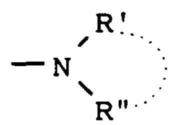

group, R' and R'', identical or different, representing independently a linear or branched alkyl group having 1 to 6 carbon atoms or representing, together with the nitrogen atom to which they are bound, a 5- to 7-membered ring which can comprise at least one other heteroatom selected from oxygen, sulphur and nitrogen, said 5- to 7-membered ring being non-substituted or substituted with an R''' group, said R''' group being selected from a linear or branched alkyl group having 1 to 6 carbon atoms, an amine group, and a nitro group; in the case where said other heteroatom is nitrogen, said nitrogen may optionally be substituted with a linear or branched alkyl group having 1 to 6 carbon atoms,

  + a methacryloyl group or an acryloyl group,
  + an epoxy group of formula :

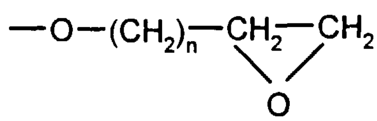

in which n = 1, 2 or 3,

- an aralkyl, aryloxyalkyl, heteroaralkyl or heteroaryloxyalkyl group, the linear or branched alkyl group having 1 to 4 carbon atoms and the aryl, aryloxy, heteroaryl, and heteroaryloxy groups having the definitions given above,

  or
- said two substituents $R_1$ and $R_2$ together form with the N-atom to which they are bound, a 5- to 7-membered ring which can comprise at least one other heteroatom selected from oxygen, sulphur and nitrogen, said 5- to 7-membered ring being non-substituted or substituted with an R''' group, said R''' group being selected from a linear or branched alkyl group having 1 to 6 carbon atoms, an amine group, and a nitro group; in the case where said other heteroatom is nitrogen, said nitrogen may optionally be substituted with a linear or branched alkyl group having 1 to 6 carbon atoms ; said two substituents $R_1$ and $R_2$, together with the N-atom to which they are bound, representing advantageously a morpholino, piperidino, piperazino, or an indolino group ;

• $R_3$, $R_4$, and $R_5$ identical or different, independently represent :

- a halogen, notably fluorine, chlorine or bromine,

- a linear or branched alkyl group having 1 to 12 carbon atoms (advantageously 1 to 6 carbon atoms),
- a cycloalkyl group having 3 to 12 carbon atoms,
- a linear or branched alkenyl or alkynyl group having 2 to 12 carbon atoms,
- a linear or branched alkoxy or alkylthio group, having 1 to 12 carbon atoms (advantageously 1 to 6 carbon atoms),
- a haloalkyl, a halocycloalkyl or haloalkoxy group corresponding respectively to the alkyl, cycloalkyl, alkoxy groups above, substituted with at least one halogen atom, notably selected from fluorine, chlorine and bromine,
- an electron-withdrawing group, such as a CN, $NO_2$ or SCN group,
- an aryl, aryloxy, heteroaryl or heteroaryloxy group having the same definition as that given above for $R_1$, $R_2$,
- an aralkyl, aryloxyalkyl, heteroaralkyl or heteroaryloxyalkyl group, the linear or branched alkyl group having 1 to 4 carbon atoms and the aryl, aryloxy, heteroaryl, and heteroaryloxy groups having the same definitions as those given above for $R_1$, $R_2$,
- an amino or amido group : $-NH_2$,- NHR, - $CONH_2$,- CONHR,

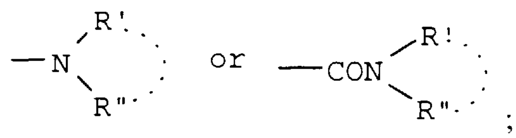

  R, R', R" having their respective definitions given above for the amine substituents of the $R_1$, $R_2$ values: aryl or heteroaryl,
- an - $OCOR_6$ or $-COOR_6$ group, $R_6$ representing a hydrogen atom, or a straight or branched alkyl group having 1 to 6 carbon atoms or a cycloalkyl group having 3 to 6 carbon atoms or a phenyl group optionally substituted with at least one of the substituents listed above for the $R_1$, $R_2$ values: aryl, aryloxy, heteroaryl or heteroaryloxy ; or
- in the case in which m is equal to or greater than 2 in $(R_4)_m$, and/or in the case in which n is equal to or greater than 2 in $(R_3)_n$, and/or in the case where p is equal to or greater than 2 in $(R_5)_p$, said radicals, being preferably borne by two adjacent carbon atoms, can combine to form at least one aromatic ring having 5 or 6 members, or an aliphatic ring having 5 to 7 members, preferably 5 or 6 members, said ring (s) comprising, optionally, at least one heteroatom selected from sulphur, oxygen and nitrogen, so as to form at least one heterocyclic ring, the latter being optionally substituted by one or more radicals, which may be identical or different, and have the same definition as given above for $R_4$ and $R_3$ ; and

• m, n, and p are independently an integer from 0 to 5.

**2.** Compounds according to claim 1 of formula (I'):

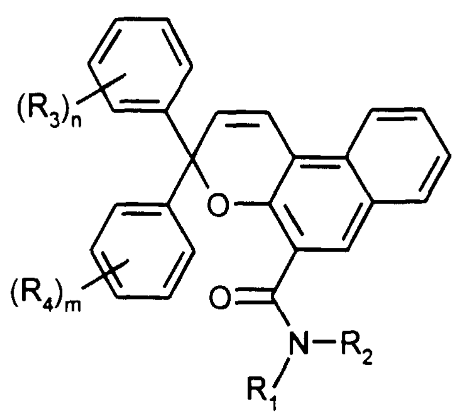

(I')

in which $R_1$, $R_2$, $R_3$, $R_4$, m, and n are as defined in claim 1 with reference to formula (I).

**3.** Compound according to claim 1 or 2 of formula (I) or (I') in which one of $R_1$ and $R_2$ is an unsubstituted or substituted aryl group, advantageously an unsubstituted or substituted phenyl group.

**4.** Compounds of formula (I) according to claim 1, which are selected from the group consisting of the following :
3,3-diphenyl-5-p-tolylcarbamoyl-3H-naphtho[2,1-b]pyran

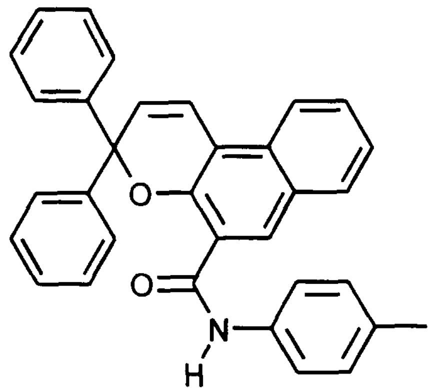

3,3-bis(2,4-dimethoxyphenyl)-5-morpholinocarbonyl-3H-naphtho[2,1-b]pyran

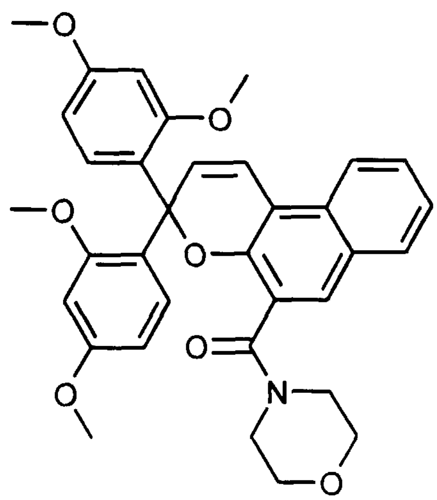

3,3-bis(4-methoxyphenyl)-5-p-tolylcarbamoyl-3H-naphtho-[2,1-b]pyran

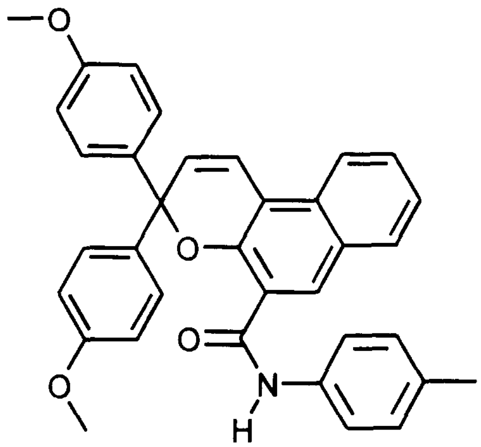

3-(4-dimethylaminophenyl)-3-(4-dimethylaminonapthyl)-5-morpholinocarbonyl-3H-naphtho[2,1-b]pyran

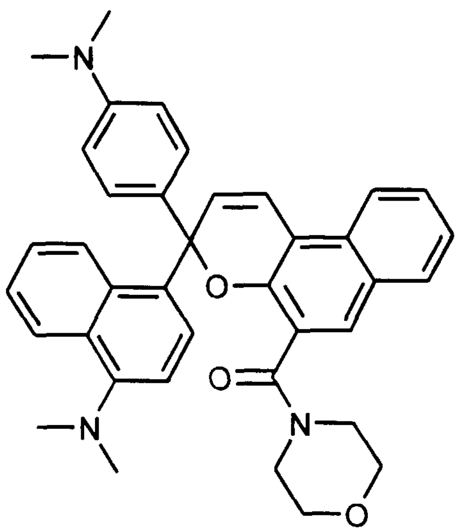

3,3-bis(2,4-dimethoxyphenyl)-5-indolinocarbonyl-3H-naphtho[2,1-b]pyran

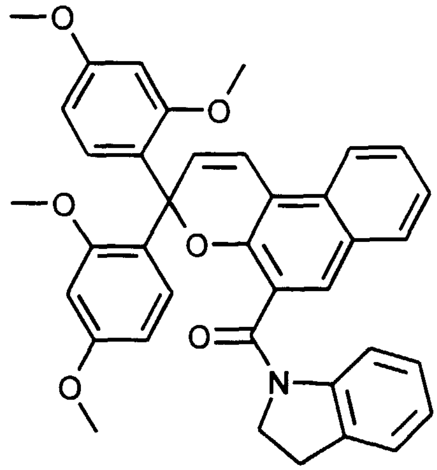

3-(2,4-dimethoxyphenyl)-3-(4-methoxyphenyl)-5-morpholinocarbonyl-3H-naphtho-[2,1-b]pyran

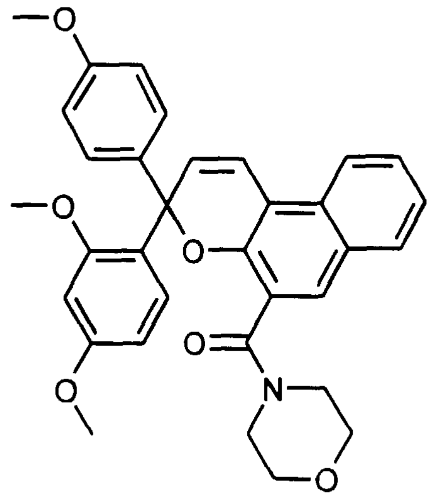

3-(2,4-dimethoxyphenyl)-3-(3,4-dimethoxyphenyl)-5-morpholinocarbonyl-3H-naphtho[2,1-b]pyran

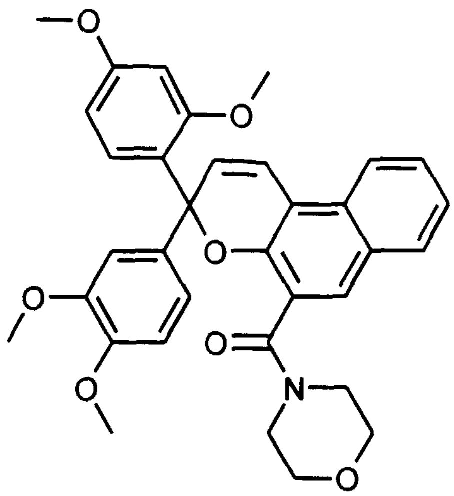

3-(2,4-dimethoxyphenyl)-3-(p-N-piperidinophenyl)-5-piperidinocarbonyl-3H-naphtho[2,1-b]pyran

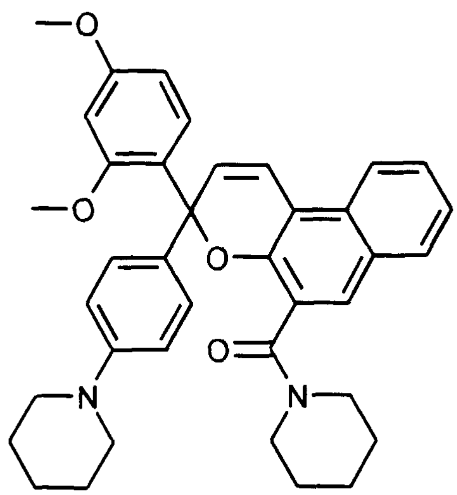

3,3-bis(2,4-dimethoxyphenyl)-5-piperidinocarbonyl-3H-naphtho[2,1-b]pyran

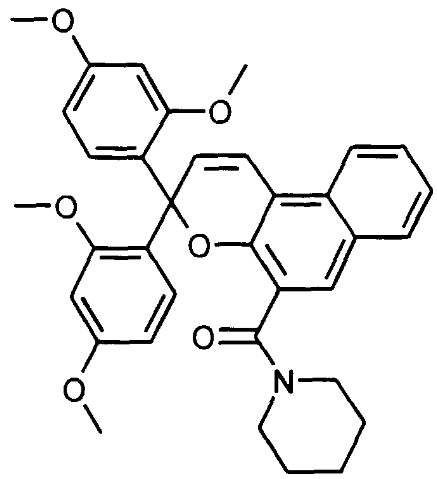

**5.** A method of preparing compounds of formula (I) according to any one of claims 1 to 4, characterised in that it comprises :

a) allowing an acetylide salt of formula (II) :

$$\equiv^{-} M^{+} \qquad \text{(II)}$$

in which M represents a metal, to react with a benzophenone of formula (III):

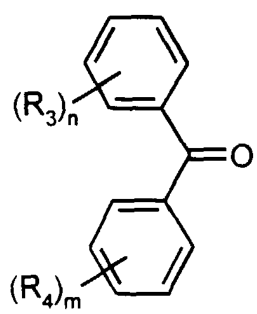

(III)

in which $R_3$, $R_4$, n, and m are as defined in any one of claims 1 to 3, to provide a propynol of formula (IV):

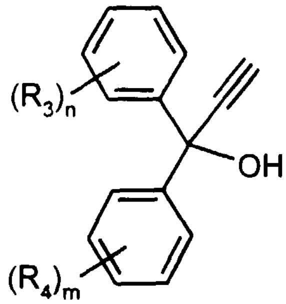

(IV)

in which $R_3$, $R_4$, n, and m are as defined in any one of claims 1 to 3, which is then allowed to react with a 2-hydroxy-3-naphthamide of formula (V) :

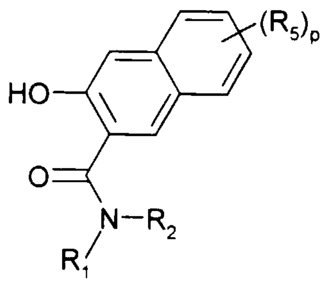

(V)

in which $R_1$, $R_2$, $R_5$, and p are as defined in any one of claims 1 to 3, to provide a compound of formula (I):

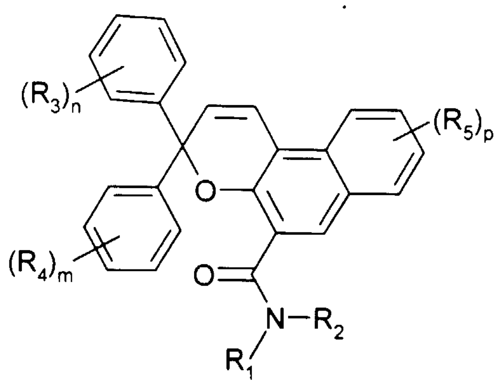

(I)

in which $R_1$, $R_2$, $R_3$, $R_4$, $R_5$, n, m , and p are as defined in any one of claims 1 to 3.

**6.** A (co)polymer and/or reticulate obtained by polymerisation and/or cross-linking and/or grafting of at least one compound according to any one of claims 1 to 4.

**7.** A photochromic compound characterised in that it consists of a compound according to any one of claims 1 to 4,

or of a mixture of at least two compounds according to any one of claims 1 to 4 or of a mixture of at least one compound according to any one of claims 1 to 4 with at least one other photochromic compound of another type and/or at least one non-photochromic colouring agent.

**8.** A photochromic composition, characterised in that it comprises :

- at least one compound according to any one of claims 1 to 4 and/or at least one (co)polymer and/or reticulate according to claim 6,
- and, optionally, at least one other photochromic compound of another type and/or at least one non-photochromic colouring agent and/or at least one stabilising agent.

**9.** A (co)polymer matrix, characterised in that it comprises :

- at least one compound according to any one of claims 1 to 4,
- and/or at least one co(polymer) and/or reticulate according to claim 6,
- and/or at least one composition according to claim 8.

**10.** The matrix according to 8, characterised in that the (co)polymer is selected from the following list :

- optionally halogenated alkyl, cycloalkyl, aryl or aralkyl poly[mono-, di-, tri-, tetra-]acrylate or poly[mono-, di-, tri-, tetra-]methacrylate or having at least one ether and/or ester and/or carbonate and/or carbamate and/or thiocarbamate and/or urea and/or amide group,
- polystyrene, polyether, polyester, polycarbonate, polycarbamate, polyepoxy, polyurea, polyurethane, polythiourethane, polysiloxane, polyacrylonitrile, polyamide, aliphatic or aromatic polyester, vinylic polymer, cellulose acetate, cellulose triacetate, cellulose acetate-propionate or polyvinylbutyral,
- copolymers of two or more types of monomers or mixtures of polymers mentioned above.

**11.** An ophthalmic or solar article comprising :

- at least one compound according to any one of claims 1 to 4,
- and/or at least one (co)polymer and/or reticulate according to claim 6,
- and/or at least one composition according to claim 8,
- and/or at least one matrix according to one of claims 9 or 10.

**12.** The article according to claim 11, characterised in that it is constituted by a lens.

**13.** A glazing and/or optical device comprising :

- at least one compound according to any one of claims 1 to 4,
- and/or at least one (co)polymer and/or reticulate according to claim 6,
- and/or at least one composition according to claim 8,
- and/or at least one matrix according to one of claims 9 or 10.

**14.** Intermediate compounds of formula (V):

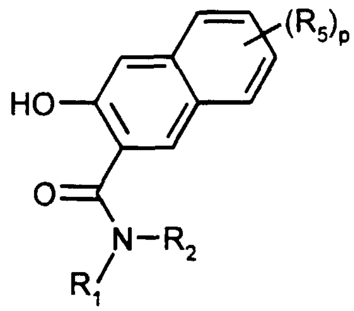

(V)

in which $R_1$, $R_2$, $R_5$, and p are as defined in claim 1.

European Patent Office

# EUROPEAN SEARCH REPORT

Application Number
EP 00 40 0796

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 5 244 602 A (B.VAN GEMERT) 14 September 1993 (1993-09-14) * claims; examples 6,7 * | 1,2,6-8 | C07D311/92 C07D405/06 C07C235/66 G02B5/23 C09K9/02 |
| X | H.SCHINDLBAUER: "REAKTIONEN MIT DIMETHYLFORMAMIDE,2." MONATSHEFTE FUR CHEMIE., vol. 100, no. 5, 1969, pages 1583-9, XP002142810 SPRINGER VERLAG. WIEN., AT ISSN: 0026-9247 * page 1588 - page 1589 * | 14 | |
| X | GB 1 496 364 A (HOECHST) 30 December 1977 (1977-12-30) * page 2 - page 4 * | 14 | |
| X | EP 0 149 158 A (POLAROID) 24 July 1985 (1985-07-24) * page 46 - page 47 * | 14 | |

TECHNICAL FIELDS SEARCHED (Int.Cl.7)

C07D
C07C
G02B

The present search report has been drawn up for all claims

| Place of search | Date of completion of the search | Examiner |
|---|---|---|
| THE HAGUE | 18 July 2000 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 40 0796

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2000

| Patent document cited in search report | Publication date | Patent family member(s) | Publication date |
|---|---|---|---|
| US 5244602 A | 14-09-1993 | AU 646125 B | 10-02-1994 |
| | | AU 9120291 A | 25-06-1992 |
| | | CA 2097606 A,C | 04-06-1992 |
| | | DE 69125395 D | 30-04-1997 |
| | | DE 69125395 T | 02-10-1997 |
| | | EP 0560898 A | 22-09-1993 |
| | | ES 2102488 T | 01-08-1997 |
| | | JP 2505357 B | 05-06-1996 |
| | | JP 6502180 T | 10-03-1994 |
| | | KR 170432 B | 18-02-1999 |
| | | MX 9102294 A | 01-06-1992 |
| | | WO 9209593 A | 11-06-1992 |
| | | US 5340857 A | 23-08-1994 |
| GB 1496364 A | 30-12-1977 | DE 2456281 A | 10-06-1976 |
| | | BE 836101 A | 28-05-1976 |
| | | CH 600033 B | 15-06-1978 |
| | | CH 1526675 A | 15-08-1977 |
| | | FR 2292801 A | 25-06-1976 |
| | | IT 1049873 B | 10-02-1981 |
| | | JP 1266274 C | 27-05-1985 |
| | | JP 51077623 A | 06-07-1976 |
| | | JP 59040851 B | 03-10-1984 |
| | | NL 7513640 A | 01-06-1976 |
| EP 149158 A | 24-07-1985 | US 4563412 A | 07-01-1986 |
| | | CA 1218057 A | 17-02-1987 |
| | | DE 3478753 D | 27-07-1989 |
| | | JP 1468699 C | 30-11-1988 |
| | | JP 60231767 A | 18-11-1985 |
| | | JP 63018179 B | 18-04-1988 |

EPO FORM P0459

For more details about this annex : see Official Journal of the European Patent Office, No. 12/82